# EUROPEAN PATENT SPECIFICATION

(11) **EP 2 480 234 B1**
(45) Date of publication and mention of the grant of the patent: **26.02.2020**
(21) Application number: 10803516.3
(22) Date of filing: 16.09.2010
(51) Int. Cl.: A61K 31/495, A61K 9/20, A61K 47/26, A61K 47/32, A61K 47/36, A61K 47/38

(54) **SUSTAINED RELEASE COMPOSITION OF RANOLAZINE**
ZUSAMMENSETZUNG AUS RANOLAZIN MIT VERZÖGERTER FREISETZUNG
COMPOSITION DE RANOLAZINE À LIBÉRATION PROLONGÉE

(30) Priority: 25.09.2009 IN 2250MU2009
(43) Date of publication of application: 01.08.2012
(73) Proprietor: Lupin Limited, Mumbai 400 055 (IN)
(72) Inventor: BHASALE, Ketan, Maharashtra (IN); NAYAK, Raghavendra, Maharashtra (IN); DAS, Subhasis, Maharashtra (IN); THOMMANDRU, Vijaya, Kumar, Maharashtra (IN)
(74) Representative: Nony
(86) International application number: PCT/IN2010/000628
(87) International publication number: WO 2011/036677

(56) References cited:
- US-A1- 2004 097 514
- DATABASE WPI Week 200836 Thomson Scientific, London, GB; AN 2008-F40218 XP002627867, & CN 101 066 254 A (BEIJING BENCAO TIANYUAN MEDICINE RES INS) 7 November 2007 (2007-11-07)

## Description

### Field of the Invention

The present invention relates to sustained release dosage form of Ranolazine or pharmaceutically acceptable salt(s), polymorph(s), solvate(s), hydrate(s), enantiomer(s) thereof which comprises a combination of at least two pH-dependent binders, wherein one binder is sodium alginate and optionally one or more pharmaceutically acceptable excipient(s).

### Background of the Invention

U.S. Pat. No. 4,567,264, discloses ranolazine, (±)-N--(2,6-dimethylphenyl)-4-[2-hydroxy-3-(2 methoxyphenoxy)-propyl]- 1-piperazineacetamide, and its pharmaceutically acceptable salts, and their use in the treatment of cardiovascular diseases, including arrhythmias, variant and exercise-induced angina, and myocardial infarction.

U.S. Pat. No. 5,472,707, discloses a high-dose oral formulation employing supercooled liquid ranolazine as a fill solution for a hard gelatin capsule or softgel.

One problem with conventional oral dosage formulations is that they are not ideally suited to ranolazine and its pharmaceutically acceptable salts, because the solubility of ranolazine is relatively high at the low pH that occurs in the stomach. Furthermore, ranolazine also has a relatively short plasma half-life. The high acid solubility property of ranolazine results in rapid drug absorption and clearance, causing large and undesirable fluctuations in plasma concentration of ranolazine and a short duration of action, thus necessitating frequent oral administration for adequate treatment.

There is therefore a need for a method for administering ranolazine in an oral dosage form once or twice daily that provides therapeutically effective plasma concentrations of ranolazine for the treatment of angina in humans.
Currently, Ranolazine is marketed as modified release tablets at the dosage of 500mg and 1gm under the brand name Ranexa®

U.S. Pat. No. 5,506,229, discloses the use of ranolazine and its pharmaceutically acceptable salts and esters for the treatment of tissues experiencing a physical or chemical insult, including cardioplegia, hypoxic or reperfusion injury to cardiac or skeletal muscle or brain tissue, and for use in transplants. Conventional oral and parenteral formulations are disclosed, including controlled release formulations. In particular, Example 7D of U.S. Pat. No. 5,506,229 describes a controlled release formulation in capsule form comprising microspheres of ranolazine and microcrystallirie cellulose coated with release controlling polymers.

US Pat. No.6,303,607 discloses a sustained release pharmaceutical dosage form including at least 50% by weight ranolazine and an admixture of at least one pH-dependent binder and at least one pH-independent binder, and wherein the peak to trough plasma ranolazine level does not exceed 3:1 over a 24 hour period.

US Pat No.20060177502A1 discloses a sustained release pharmaceutical formulation comprising: less than 50% ranolazine; a pH dependent binder; a pH independent binder; and one or more pharmaceutically acceptable excipients.

However, there are alternate means by which we can design a sustained release dosage form of Ranolazine or pharmaceutically acceptable salt(s), polymorph(s), solvate(s), hydrate(s), enantiomer(s).

Surprisingly, it has been found that sustained release dosage form of ranolazine can be prepared using combination of at least two pH dependent binders, wherein one binder is sodium alginate, that is bioequivalent to the marketed formulation and provide the appropriate plasma levels of ranolazine that are necessary for the treatment of angina and other cardiovascular diseases.

### Objects Of The Invention

The object of the present invention is a sustained release pharmaceutical dosage form comprising a therapeutically effective amount of ranolazine or pharmaceutically acceptable salt(s), polymorph(s), solvate(s), hydrate(s), enantiomer(s), a combination of at least two pH-dependent binders, wherein one binder is sodium alginate, and optionally one or more pharmaceutically acceptable excipients (s).

Another object of the invention is a sustained release pharmaceutical dosage form comprising a therapeutically effective amount of ranolazine or pharmaceutically acceptable salt(s), polymorph(s), solvate(s), hydrate(s), enantiomer(s), combination of atleast two pH dependent binders, wherein one binder is sodium alginate, and optionally one or more pharmaceutically acceptable excipient(s) wherein ranolazine is atleast about 50% of the core weight.

Another object of the invention is a sustained release pharmaceutical dosage form comprising a therapeutically effective amount of ranolazine or pharmaceutically acceptable salt(s), polymorph(s), solvate(s), hydrate(s), enantiomer(s), combination of atleast two pH dependent binders, wherein one binder is sodium alginate, and optionally one or more pharmaceutically acceptable excipient(s) characterized in that the sustained release pharmaceutical composition of invention is bioequivalent to marketed formulation.

Another object of the invention is a process for preparing sustained release dosage form ranolazine, wherein the process comprises the steps of i) blending Ranolazine or a pharmaceutically acceptable salt(s), polymorph(s), solvate(s), hydrate(s), enantiomer(s) thereof with combination of at least two pH dependent binders, wherein one binder is sodium alginate, optionally one or more pharmaceutically acceptable excipient(s) ii) granulating the dry blend with granulating liquid iii) drying the wet mass to obtain the granules iv)mixing the granules with other excipient(s) and v) compressing the granules to form the solid oral dosage.

Another object of the invention is a sustained release pharmaceutical dosage form comprising a therapeutically effective amount of ranolazine or pharmaceutically acceptable salt(s), polymorph(s), solvate(s), hydrate(s), enantiomer(s), combination of at least two pH dependent polymers, wherein one polymer is sodium alginate, and optionally one or more pharmaceutically acceptable excipient(s) wherein about 20% to about 40% of said ranolazine is released after 2 hours; from about 45% to about 65% of said ranolazine is released after 8 hours; not less than about 70% of said ranolazine is released after 24 hours.

### Detailed Description Of The Invention

"Ranolazine" is (±)-N-(2,6-dimethylphenyl)-4-[2-hydroxy-3-(2-methoxyphenoxy) propyl]-1-piperazine-acetamide, or its enantiomers (R)-(+)-N-(2,6-dimethylphenyl)-4-[2-hydroxy-3-(2-methoxypheno-xy)-propyl]-1-piperazineacetamide, and (S)-(-)-N-(2,6-dimethylphenyl)-4-[2- -hydroxy-3-(2-methoxyphenoxy)-propyl]-1-piperazineacetamide and their pharmaceutically acceptable salts, and mixtures thereof.

As used herein, "pharmaceutically acceptable salts" refer to derivatives of the ranolazine wherein ranolazine is modified by reacting it with an acid or base as needed to form an ionically bound pair. Examples of pharmaceutically acceptable salts include conventional non- toxic salts or the quaternary ammonium salt of the parent compound formed, for example, from non-toxic inorganic or organic acids. Suitable non-toxic salts include those derived from inorganic acids such as hydrochloric, hydrobromic, sulfuric, sulfamic, phosphoric, nitric, and others known to those of ordinary skill in the art. The salts prepared from organic acids such as amino acids, acetic, propionic, succinic, glycolic, stearic, lactic, malic, tartaric, citric, ascorbic, pamoic, maleic, hydroxymaleic, phenyl acetic, benzoic, salicylic, sulfanilic, fumaric, oxalic, isethionic, and others known to those of ordinarily skilled in the art.

The term "sustained release" as used herein in relation to the dosage form means which is not immediate release and is taken to encompass controlled release, prolonged release, timed release, retarded release, extended release and delayed release. Sustained release can be used interchangeably with prolonged release, programmed release, timed release, extended release, controlled release and other such dosage forms.

The "dosage form" according to the present invention include but is not limited to tablets, pellets, beads, granules, capsules, microcapsules and tablets in capsules.

"Therapeutically effective amount" means that the amount of active agent, which halts or reduces the progress of the condition being treated or which otherwise completely or partly cures or acts palliatively on the condition. A person skilled in the art can easily determine such an amount by routine experimentation and with an undue burden.

By "pharmaceutically acceptable" is meant a carrier comprised of a material that is not biologically or otherwise undesirable.

"Optionally" means that the subsequently described event or circumstance may or may not occur, and that the description includes instances where the event or circumstance occurs and instances in which it does not. For example, "optionally pharmaceutical excipients" indicates that a formulation so described may or may not include pharmaceutical excipients other than those specifically stated to be present, and that the formulation so described includes instances in which the optional excipients are present and instances in which they are not.

The invention relates to sustained release dosage form of ranolazine or pharmaceutically acceptable salt(s), polymorph(s), solvate(s), hydrate(s), enantiomer(s) thereof which comprises a combination of at least two pH-dependent binders, wherein one binder is sodium alginate and optionally one or more pharmaceutically acceptable excipient(s).

Ranolazine is relatively insoluble in aqueous solutions having a pH above about 6.5, while the solubility begins to increase dramatically below about pH 6, therefore it is very difficult to prepare a sustained release dosage form of ranolazine wherein the drug is released throughout the gastrointestinal tract (GIT).

To provide a sustained release dosage form of ranolazine wherein the drug is released throughout the GIT, a combination of at least two pH-dependent binders, wherein one binder is sodium alginate, are chosen to control the dissolution rate of the ranolazine so that the dosage form releases ranolazine slowly and continuously as it passes through the stomach and gastrointestinal tract. The dissolution control capacity of the pH-dependent binder(s) is particularly important in a sustained release ranolazine formulation because a sustained release formulation that contains sufficient ranolazine may cause untoward side effects if the ranolazine is released too rapidly ("dose-dumping").

Accordingly, binders suitable for use in this invention are materials which include but not limited to carbopol, large number of phthalic acid derivatives such as cellulose phthalate, cellulose acetate phthalate, polyvinylacetate phthalate, polyvinylpyrrolidone phthalate, hydroxypropylmethylcellulose phthalate and copolymers of methacrylic acid and methacrylic or acrylic acid esters. Particularly preferred binders for use in this invention are the commercially available copolymers of methacrylic acid and a methacrylic or acrylic acid ester, for example the Eudragit polymers, particularly the Eudragit L series such as Eudragit L30D and Eudragit L100/55, sold by the Rohm and Haas Company. Eudragit L100-55 and Eudragit L30D (a dispersion of 30% Eudragit L powder in water) and alginic acid. Preferably alginic acid will take the form of an alkali metal salt such as sodium alginate or potassium alginate or ammonium alginate, and preferably sodium alginate. Preferably the combination of at least two pH-dependent binders according to the present invention is sodium alginate and Eudragit.

Proper selection of combination of pH dependent binders in the dosage form helps in sustaining the release of Ranolazine throughout the GIT. Partial neutralization of the binder facilitates the conversion of the binder into latex like film which forms around the individual ranolazine granules. Accordingly, the type and the quantity of the pH-dependent binder and amount of the partial neutralization composition are chosen to closely control the rate of dissolution of the ranolazine from the formulation.

The amount of active present in the dosage form according to the present invention is in the range of 45 to 100% and more preferably 70 to 80%.

Pharmaceutically acceptable excipient(s) include but are not limited to diluents, lubricants, disintegrants, glidants and surface-active agents.

The amount of excipient employed will depend upon how much active agent is to be used. One excipient can perform more than one function.

Fillers or diluents, which include, but are not limited to confectioner's sugar, compressible sugar, dextrates, dextrin, dextrose, fructose, lactitol, mannitol, sucrose, starch, lactose, xylitol, sorbitol, talc, microcrystalline cellulose, calcium carbonate, calcium phosphate dibasic or tribasic, calcium sulphate, and the like can be used.

Lubricants may be selected from, but are not limited to, those conventionally known in the art such as Mg, Al or Ca or Zn stearate, polyethylene glycol, glyceryl behenate, mineral oil, sodium stearyl fumarate, stearic acid, hydrogenated vegetable oil and talc.

Glidants include, but are not limited to, silicon dioxide; magnesium trisilicate, powdered cellulose, starch, talc and tribasic calcium phosphate, calcium silicate, magnesium silicate, colloidal silicon dioxide, silicon hydrogel and other materials known to one of ordinary skill in the art.

The present compositions may optionally contain disintegrants which include but are not limited to starches; clays; celluloses; alginates; gums; cross-linked polymers, e.g., cross-linked polyvinyl pyrrolidone or crospovidone, e.g., POLYPLASDONE XL, cross-linked sodium carboxymethylcellulose or croscarmellose sodium, e.g., AC-DI-SOL from FMC; and cross-linked calcium carboxymethylcellulose; and guar gum.

The present compositions may optionally contain a surface-active agent. The preferred agent is copolymers composed of a central hydrophobic chain of polyoxypropylene (poly (propylene oxide)) and polyoxyethylene (poly (ethylene oxide)) that is well known as poloxamer. However, other agents may also be employed such as dioctyl sodium sulfosuccinate (DSS), triethanolamine, sodium lauryl sulphate (SLS), polyoxyethylene sorbitan and poloxalkol derivatives, quaternary ammonium salts or other pharmaceutically acceptable surface-active agents known to one ordinary skilled in the art.

The pharmaceutical composition of the invention can be formed by various methods known in the art such as by dry granulation, wet granulation, melt granulation, direct compression, double compression, extrusion spheronization, layering and the like. Sustained release pharmaceutical dosage form according to the present invention are manufactured preferably as per the following procedure:
i) Blending the active agent, pH dependent binders, wherein one binder is sodium alginate, one or more pharmaceutically acceptable excipient(s),
ii) Subjecting the blend to granulation.
iii) Drying and sieving the granulated blend to obtain granules
iv) Blending the granules obtained in the above step with extragranular excipient(s)
v) Compressing the blend of step (iv) to form the solid oral dosage form.

The pharmaceutical dosage forms of the invention may further be coated. The coating may be a functional or non functional coating. The preferred coating of this invention is comprised of a commercial film-coating product designed for aqueous film coating containing the water-soluble, film-forming resin, such a product is commercially available under the trade name Opadry White (Colorcon, West Point, Pa.).

These coating comprises one or more excipients selected from the group comprising coating agents, opacifiers, fillers, polishing agents, colouring agents, antitacking agents and the like.

The examples given below are illustrative embodiments of the invention and are merely exemplary.

**Example 1**

| **Sr.No** | **Ingredients** | **%w/w** |
|---|---|---|
| | **Core** | |
| 1 | Ranolazine | 75.75 |
| 2. | Microcrystalline Cellulose | 7.78 |
| 3 | Lactose | 3.15 |
| 4 | Sodium alginate | 1.99 |
| 5 | Eudragit | 9.84 |
| 6 | Sodium hydroxide | 0.20 |
| 7 | Purified Water | q.s |
| 8 | Colloidal silicone dioxide | 0.5 |
| 9 | Magnesium stearate | 0.76 |

| | **Coating** | |
|---|---|---|
| | Film coating | q.s |

### Brief manufacturing process

**1. Sifting:** Sift Ranolazine, MCC, lactose, Sodium alginate and Eudragit through a specific mesh and mix.
**2. Granulation:**
   Granulate the dry blend of above step with aqueous solution of NaOH to get granules of desired consistency
**3. Drying:** Dry the granules in rapid drier.
**4. Sizing and lubrication:**
   Pass the dried granules through a particular mesh.
   Sift extragranular colloidal silicone dioxide and mix with dried granules.
   Sift magnesium stearate through suitable mesh and lubricate with blend.
**5. Compression:** Compress the lubricated blend of above step using appropriate tools.
**6. Coating:** Film coat the compressed tablets.

**Example 2 (not claimed)**

| **Sr.No** | **Ingredients** | **%w/w** |
|---|---|---|
| | **Core** | |
| 1 | Ranolazine | 75.75 |
| 2. | Microcrystalline Cellulose | 4.8 |
| 3 | Lactose | 3.15 |
| 4 | Carbopol | 5 |
| 5 | Eudragit | 9.85 |
| 6 | Sodium hydroxide | 0.205 |
| 7 | Purified Water | q.s |
| 8 | Colloidal silicon dioxide | 0.45 |
| 9 | Magnesium stearate | 0.75 |

| | **Coating** | |
|---|---|---|
| | Film coating | q.s |

### Brief manufacturing process

**1. Sifting:**
   Sift Ranolazine, MCC, lactose, Carbopol and Eudragit through a specific mesh and mix.
**2. Granulation:**
   Granulate the dry blend of above step with aqueous solution of NaOH to get granules of desired consistency
**3. Drying:** Dry the granules in rapid drier.
**4. Sizing and lubrication:**
   Pass the dried granules through a particular mesh.
   Sift extragranular colloidal silicone dioxide and mix with dried granules.
   Sift magnesium stearate through suitable mesh and lubricate with blend.
**5. Compression:** Compress the lubricated blend of above step using appropriate tools.
**6. Coating:** Film coat the compressed tablets.

### In-vitro dissolution details

The dosage form of the invention have a prolonged in vitro release rate. The in vitro test used to measure release rate of the active agent from a formulation is: A solution of 900 ml of a 0.1N HCl was placed in an apparatus capable of agitation. The apparatus contained a paddle and rotated at a speed of 50 rpm. The tablet formulation was placed in the apparatus and dissolution was periodically measured. The in vitro dissolution studies of Example 1 is as follows:

| **Time (Hr)** | **% Drug Release** |
|---|---|
| 2 | about 20- about 40% |
| 4 | about 30 - about 50% |
| 8 | about 45 - about 65% |
| 12 | about 55 - about 75% |
| 16 | about 65 - about 90% |
| 20 | about 70 -about 95% |
| 24 | not less than about 70% |

### In -vivo Bioequivalence Study

Open Label, balanced, randomized, two-treatment, two-sequence, two-periods single dose two-way crossover relative oral bioavailability study of Ranolazine sustained release tablet 1000mg(T) of Lupin Limited was compared with that of Ranexa 1000mg(R) in 12 healthy adult male human subjects under fasting and fed conditions.

**Table 1: Comparative pharmacokinetic parameters of present invention vs Ranexa (1000 mg) (Fed study)**

| | ***AUC (0-t)*** | ***AUC (0-*∞*)*** | ***Cmax*** |
|---|---|---|---|
| | **ng.hr/ml** | ***ng.hr*/*ml*** | ***ng*/*ml*** |
| ***T*/*R*** | *112.99* | *93.78* | *100.60* |

**Table 2: Comparative pharmacokinetic parameters of present invention vs Ranexa (1000 mg) (Fasting study)**

| | ***AUC (0-t)*** | ***AUC (0-*∞*)*** | ***Cmax*** |
|---|---|---|---|
| | ***ng.hr*/*ml*** | ***ng.hr*/*ml*** | ***ng*/*ml*** |
| ***T*/*R*** | *108.13* | *99.59* | *99.04* |

## Claims

1. A sustained release pharmaceutical dosage form comprising a therapeutically effective amount of ranolazine or pharmaceutically acceptable salt(s), polymorph(s), solvate(s), hydrate(s), enantiomer(s) thereof; a combination of at least two pH-dependent binders wherein one binder is sodium alginate and optionally one or more pharmaceutically acceptable excipients (s).

2. A sustained release pharmaceutical dosage form as in claim 1, wherein the pH dependent binders are selected from the group comprising carbopol, phthalic acid derivatives, copolymers of methacrylic acid and methacrylic or acrylic acid esters, and alginic acid.

3. A sustained release pharmaceutical dosage form as in claim 1, wherein pharmaceutically acceptable excipients are selected from the group comprising diluents, lubricants, surfactants and glidants.

4. A sustained release pharmaceutical dosage form as in claim 1 is wherein the dosage form is selected from group comprising granules, capsules, tablet, pellets, minitablets, microcapsules, tablet in capsules, granules in capsules, and pellets in capsules.

5. A sustained release pharmaceutical dosage form according to claim 1 wherein ranolazine is at least about 50% of the core weight.

6. A sustained release pharmaceutical dosage form according to claim 1 wherein about 20% to about 40% of said ranolazine is released after 2 hours; from about 45% to about 65% of said ranolazine is released after 8 hours; not less than about 70% of said ranolazine is released after 24 hours.

7. A process for preparing sustained release dosage form ranolazine, wherein the process comprises the steps of i) blending Ranolazine or a pharmaceutically acceptable salt(s), polymorph(s), solvate(s), hydrate(s), enantiomer(s) thereof; a combination of at least two pH dependent binders wherein one binder is sodium alginate and optionally one or more pharmaceutically acceptable excipient(s) ii) granulating the dry blend with granulating liquid iii) drying the wet mass to obtain the granules iv)mixing the granules with other excipient(s) and v) compressing the granules to form the solid oral dosage.

## Patentansprüche

1. Pharmazeutische Dosierungsform mit gleichmäßig hinhaltender Wirkstofffreigabe, umfassend eine therapeutisch wirksame Menge von Ranolazin oder eines oder mehrerer pharmazeutisch unbedenklicher Salze, Polymorphe, Solvate, Hydrate, Enantiomere davon, eine Kombination von mindestens zwei pH-abhängigen Bindemitteln, wobei es sich bei einem Bindemittel um Natriumalginat handelt, und gegebenenfalls einen oder mehrere pharmazeutisch unbedenkliche Hilfsstoffe.

2. Pharmazeutische Dosierungsform mit gleichmäßig hinhaltender Wirkstofffreigabe nach Anspruch 1, wobei die pH-abhängigen Bindemittel aus der Gruppe umfassend Carbopol, Phthalsäurederivate, Copolymere von Methacrylsäure und Methacrylsäure- oder Acrylsäureestern und Alginsäure ausgewählt sind.

3. Pharmazeutische Dosierungsform mit gleichmäßig hinhaltender Wirkstofffreigabe nach Anspruch 1, wobei die pharmazeutisch unbedenklichen Hilfsstoffe aus der Gruppe umfassend Verdünnungsmittel, Schmiermittel, Tenside und Gleitmittel ausgewählt sind.

4. Pharmazeutische Dosierungsform mit gleichmäßig hinhaltender Wirkstofffreigabe nach Anspruch 1, wobei die Dosierungsform aus der Gruppe umfassend Granulate, Kapseln, Tabletten, Pellets, Minitabletten, Mikrokapseln, Tabletten in Kapseln, Granulate in Kapseln und Pellets in Kapseln ausgewählt ist.

5. Pharmazeutische Dosierungsform mit gleichmäßig hinhaltender Wirkstofffreigabe nach Anspruch 1, wobei Ranolazin mindestens etwa 50 Gew.-% des Kerngewichts ausmacht.

6. Pharmazeutische Dosierungsform mit gleichmäßig hinhaltender Wirkstofffreigabe nach Anspruch 1, wobei nach 2 Stunden etwa 20 % bis etwa 40 % des Ranolazins freigesetzt sind, nach 8 Stunden etwa 45 % bis etwa 65 % des Ranolazins freigesetzt sind, nach 24 Stunden nicht weniger als etwa 70 % des Ranolazins freigesetzt sind.

7. Verfahren zur Herstellung einer Dosierungsform von Ranolazin mit gleichmäßig hinhaltender Wirkstofffreigabe wobei das Verfahren die Schritte i) Mischen von Ranolazin oder einem oder mehreren pharmazeutisch unbedenklichen Salzen, Polymorphen, Solvaten, Hydraten, Enantiomeren davon, einer Kombination von mindestens zwei pH-abhängigen Bindemitteln, wobei es sich bei einem Bindemittel um Natriumalginat handelt, und gegebenenfalls einem oder mehreren pharmazeutisch unbedenklichen Hilfsstoffen, ii) Granulieren der trockenen Mischung mit Granulierflüssigkeit, iii) Trocknen der feuchten Masse zum Erhalt des Granulats, iv) Mischen des Granulats mit einem oder mehreren anderen Hilfsstoffen und v) Verpressen des Granulats zur Bildung der festen oralen Dosierung umfasst.

## Revendications

1. Forme pharmaceutique à libération prolongée comprenant une quantité thérapeutiquement efficace de ranolazine ou des sel(s) pharmaceutiquement acceptable(s), polymorphe(s), solvate(s), hydrate(s), énantiomère(s) de celle-ci ; une combinaison d'au moins deux liants dépendants du pH où un liant est l'alginate de sodium et facultativement un ou plusieurs excipient(s) pharmaceutiquement acceptable(s).

2. Forme pharmaceutique à libération prolongée selon la revendication 1, dans laquelle les liants dépendants du pH sont choisis dans le groupe comprenant Carbopol, des dérivés d'acide phtalique, des copolymères d'acide méthacrylique et d'esters d'acide méthacrylique ou acrylique, et l'acide alginique.

3. Forme pharmaceutique à libération prolongée selon la revendication 1, dans laquelle les excipients pharmaceutiquement acceptables sont choisis dans le groupe comprenant des diluants, des lubrifiants, des tensioactifs et des agents glissants.

4. Forme pharmaceutique à libération prolongée selon la revendication 1, la forme pharmaceutique étant choisie dans le groupe comprenant des granules, des capsules, un comprimé, des pastilles, des minicomprimés, des microcapsules, un comprimé dans des capsules, des granules dans des capsules, et des pastilles dans des capsules.

5. Forme pharmaceutique à libération prolongée selon la revendication 1, dans laquelle la ranolazine constitue au moins environ 50 % du poids du noyau.

6. Forme pharmaceutique à libération prolongée selon la revendication 1, dans laquelle environ 20 % à environ 40 % de ladite ranolazine est libérée après 2 heures ; d'environ 45 % à environ 65 % de ladite ranolazine est libérée après 8 heures ; pas moins d'environ 70 % de ladite ranolazine est libérée après 24 heures.

7. Procédé de préparation d'une forme pharmaceutique à libération prolongée de ranolazine, le procédé comprenant les étapes de i) mélange de ranolazine ou de sel(s) pharmaceutiquement acceptable(s), polymorphe(s), solvate(s), hydrate(s), énantiomère(s) de celle-ci ; une combinaison d'au moins deux liants dépendants du pH, où un liant est l'alginate de sodium et facultativement un ou plusieurs excipient(s) pharmaceutiquement acceptable(s) ii) granulation du mélange sec avec un liquide de granulation iii) séchage de la masse humide pour obtenir les granules iv) mélange des granules avec un ou plusieurs autre(s) excipient(s) et v) compression des granules pour former la forme pharmaceutique orale solide.
